# EUROPEAN PATENT SPECIFICATION

(11) **EP 2 491 897 B1**
(45) Date of publication and mention of the grant of the patent: **07.03.2018**
(21) Application number: 11382052.6
(22) Date of filing: 28.02.2011
(51) Int. Cl.: A61F 5/41

(54) **Multifunctional penile extender**
Multifunktioneller Penisstrecker
Dispositif d'allongement de pénis multifonctionnel

(43) Date of publication of application: 29.08.2012
(73) Proprietor: Gomez de Diego, Eduardo Antonio, 28231 Las Rozas (Madrid) (ES)
(72) Inventor: Gomez de Diego, Eduardo Antonio, 28231 Las Rozas (Madrid) (ES)
(74) Representative: Temiño Ceniceros, Ignacio

(56) References cited:
- EP-A1- 1 473 000
- EP-A1- 1 878 410
- WO-A2-2010/042813
- DE-A1- 19 618 352
- US-A1- 2009 247 377

## Description

The object of the multifunctional penile extender is a system that by combining two interrelated devices, such as silicone band and a support for the top of the penis; wherein with some novel features, it is possible to improve the comfortability of use and increase the functions and indications of use for the system against the systems described in the state of the art.

### STATE OF THE PRIOR ART

The document EP1473000 describes a device for the treatment of genital hypoplasia comprising: an external genital fixation to ensure the glans to the device; an internal fixation system and a dynamometric system for tissue expansion and that is characterized in that comprises a silicone ring placed severally attached to a superior support and also comprises a protection system of the glans.

This document describes a penis enlargement system by traction and that, in order to improve the comfort of use, comprises a conventional silicone band in combination with a protection element, typically a pad or similar. However, the pad is a separate element of the band, which deteriorates over time, and as the wear occurs, exerts its function worse. Similarly, the silicone ring is essentially cylindrical offering a high pressure per cm² when transmits the traction force of these systems over the contact area on the penis. Clearly, as the wear in the pads occurs, the feeling of discomfort in the use of the device will increase, causing frictions and irritation in the member. At this point, it must be had in mind that these types of devices, in order to be effective, must be placed an average of 8 hours daily in the penis, being this period large enough so that the pad, always in the same position and pressured by the silicone ring, adopts an undesirable form, sufficiently tight enough on the contact zone of the silicone ring to cause a friction or irritation balano-preputial sulcus. These problems are generally applicable to any system that uses bands or rings made of silicone or similar material, whose structure is essentially cylindrical, as the document EP 2153805, WO9728764 and WO9626691.

Another present document in the state of the art is ES2281639T3 which describes a supporting ring of the penis that is substantially flat and exerts its function as a clip on the area immediately below the glans. More specifically, that supporting ring is described as a fixing means the formed by an essentially cylindrical component and which comprises, wholly or partially, elastically the respective part of the body with a retaining clip, which, after placement of the fixing means can be blocked into the side on the periphery of the stretching rod. This system, as mentioned, is similar to the used by the ring clips. Similarly, its constitution completely flat as a whole implies that for its union with the support of the penis, it depends on the position of the flange. At this point it is noted that a cylindrical adjustable union at any point by the user is the best solution. In the described system, the comfort depends on the setting of the flanges. It is a known fact that each member is of a certain thickness and does not respond to default patterns, then for a perfect fit, the use of preset flanges is not a universal optimal solution, but there may be users where their perfect fit is between two adjacent positions of the flanges. Also the transmission of traction force is not performed on the whole surface of the clip but only in its upper edge. Said upper edge contacts with the corona of the glans, where it abuts, and transmits the traction force with a high pressure per cm² over the contact area with the penis. Another document that describes a similar system is the EP 1878410.

All documents mentioned are relates to penis enlargement devices or systems by traction and essentially consist of a circular base, where the penis is inserted, and where said base is housed in the area close to the lower base of the penis. From said base ring emanate parallel both metal rods with different particularities and functions in terms of traction, which are joined at the top by a bridge element, which is also support of the penis at its top, in contact with the base of the glans. This bridge or support element generally has the particularity of being of plastic material and including anchoring means for a silicone band, usually a couple of through holes adapted to accommodate the ends of said band, as well as anchoring or adjustment means for of the band to the back of said bridge element. This configuration lacks flexibility, which in turn results in lack of comfort in daily use. While the shape of the bridge, "U-shaped", so that the penis rests in a concave region of the element, is known in the state of the art, in all reviewed cases, the through holes are symmetrical in relation to the vertical axis of symmetry of the element, since its primary function in all cases is to exercise the penis traction. However, and as it has been reviewed, in order to improve comfort of use must be taken into account that each member has a specific shape, which in some cases may results in disorder and must be taken into account to improve such comfort. Thus, in a device of this type, a member with certain degree of lateral deviation will suffer much more pressure from one side than the other, resulting obviously in a clear lack of comfort.

There are also treatment limitations due to be designed said piece only with two holes and also be both located in a short distance and at the top of the piece. Said limitation always makes that the traction force will be exerted on a vertical axis parallel to the shaft of the penis. Said axis of this parallel traction force would not be indicated when the penis presents a deviation, since the force applied on the side of the curvature must be greater than the force applied on the side with the lesser curvature. This lack of adaptation to the axis of the curved penises makes that these previous systems have not solved the problem of treatment of curved penises which have a deviated axis, being the previous systems clearly insufficient to treat these cases.

Also these prior systems have the limitation of being the two holes from the upper piece located on top of the piece. This situation limits the possibilities for the treatment of micropenis whose length is not enough to be embraced by the silicone band, because they are located on top of said piece.

Furthermore the small distance between the holes from the upper piece limits and makes impossible the use of these systems in penises that have been recently operated since the bandage substantially increases the thickness of the penis bandaged, not being able to embrace the penis by silicone band in these cases where it is necessary to apply a traction force after urological surgeries involving a penile shortening as a side effect. Document WO 2010/042813 discloses a penile compression device comprising a band having a flat central portion. Document DE 19618352 is considered the closest prior art and discloses a penis traction system having a concave penis support device with through holes and a penis fastening device consisting of a flexible, elastic material. Document US 2009/247377 discloses a device similar to DE 19618352.

### EXPLANATION OF THE INVENTION

The object of this invention is a multifunctional penile extender configured on a penis traction system which essentially improves the comfort in use and allows its therapeutic use in the penis curvature correction in different directions (curvatures to up, down, left and right), deviations of the penis to right and left, treatment of micropenis, treatment of post-surgical penile retraction of urological surgeries of penis, prostate and bladder, and the penis enlargement and thickening.

The object of the invention is solved by a penis traction system according to independent claim 1. Practical embodiments of the invention are disclosed in dependent claims 2 to 5. Furthermore, the object of the invention is solved by a multifunctional penile extender according to independent claim 6.

This system is essentially a penis traction system made up of a base adaptable to the user body and joined by extensible rods to a plastic support or support device, and where said system has the particularity of combining the use of two devices interrelated mutually: (a) a first penis support device comprising a plurality of through holes (six in the preferred embodiment) symmetrically distributed, by which is inserted the second penis fastening device, and where depending on which pair of holes are used to support the penis, will be used to correct a pathology or other associated to the penis shape; and (b) a second penis fastening device consisting essentially of a silicone band with its central region flattened adapting a girth which is the third part of the band diameter and its width that is three times.

The shape of the central region of the silicone band increases the contact surface of the penis, logically decreasing the pressure per cm2 and, therefore, ensuring greater effectiveness in its use than the silicone bands described in the state of the art, in general of cylindrical section.

### BRIEF DESCRIPTION OF THE DRAWINGS

FIG. 1 shows a plant, elevation and side view of the penis fastening device that is part of the system object of the invention.
FIG. 2 shows a perspective view of penis support device that is part of the system object of the invention.
FIG. 3 shows schematically the use of the object of the invention in the treatment of the curvature to the left of the penis, in front and back view.
FIG. 4 shows schematically the use of the system object of the invention in the treatment of the curvature to the right of the penis, in front and back view.
FIG. 5 shows schematically the use of the system object of the invention in the treatment of upward curvature of the penis.
FIG. 6 shows schematically the use of the system object of the invention in the treatment of downward curvature of the penis.
FIG. 7 shows schematically the use of the system object of the invention in the treatment of deviation to the left of the penis.
FIG. 8 shows schematically the use of the system object of the invention in the treatment of deviation to the right of the penis.
FIG. 9 shows schematically the use of the system object of the invention to prevent scar retraction, in front and back view.
FIG. 10 shows schematically the use of the system object of the invention for enlarging and treating of micro-penises.

### DETAILED DESCRIPTION OF THE EMBODIMENTS

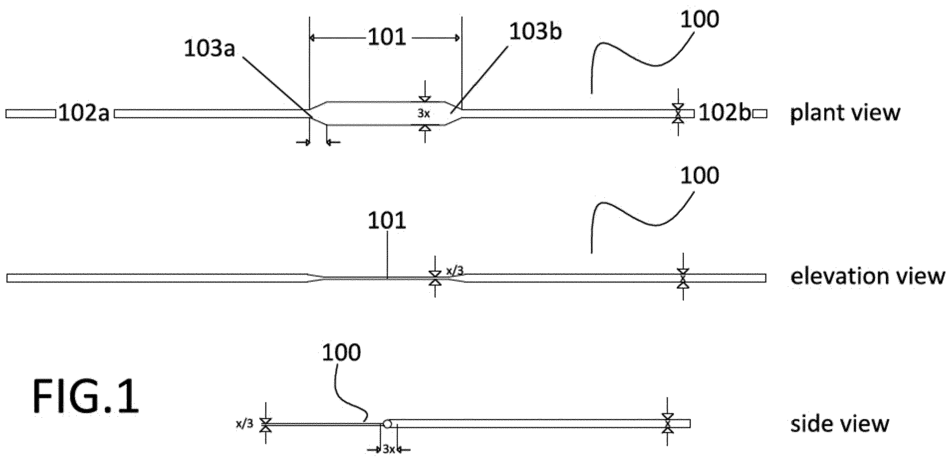

As can be seen in **FIG. 1****,** the penis fastening device (100) is characterized in that consists of a band of flexible, elastic material, in this particular embodiment silicone, having a substantially flat central region (101) and substantially cylindrical ends (102a, 102b) having a diameter (x) wherein the central region (101) has: a thickness approximately equal to one third of the diameter (x) and a width approximately equal to three times the cited diameter (x).

This structure is made up, on the one hand, to improve ease of comfort of use against the purely cylindrical elements, as has already been indicated, and on the other hand, facilitate the support and fixation of the penis, improving the comfort of use against the essentially flat devices. Similarly, the relative measures between flat region (101) and cylindrical ends (102a, 102b) are defined by the optimal distribution of pressure per cm² in the area where the device (100) will be placed, in contact with the lower base of the glans.

The length of the central region (101) further is approximately equal to one fifth of the total length of the penis fastening device (100), being established a transition area (103a, 103b) between the cylindrical ends (102a, 102b) and the central region (101), wherein each transition zone (103a, 103b) has a length approximately equal to one tenth of the total length of the central region (101).

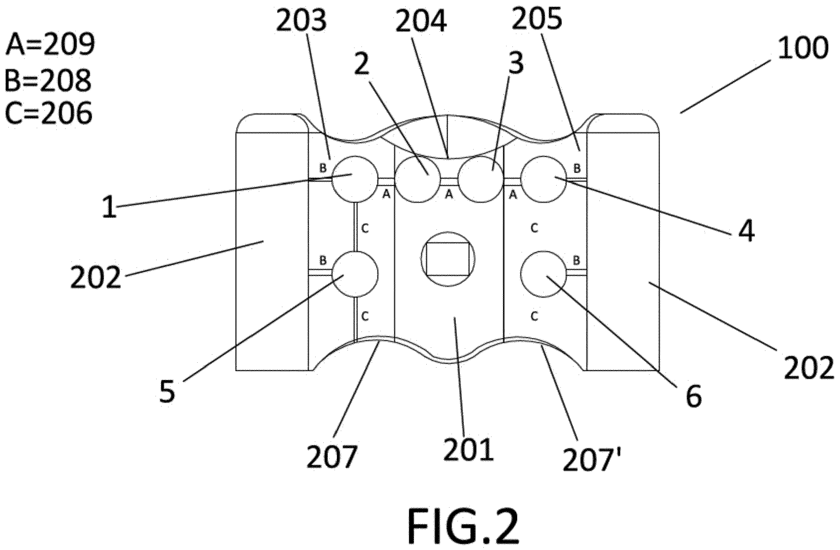

**FIG. 2** shows a view of the penis support device (200) comprising an essentially U-shaped body (201) made of plastic material, said body (201) comprising attachment means (202) configured to attach the U-shaped body (201) to metal arms which are extended in parallel with each other and longitudinally regarding to the penis.

Said device (200) is characterized in that the body (201) in its concave frontal area is defined by three regions (203, 204, 205) wherein: a first region (203) and a third region (205) are extreme regions in contact with the attachment means (202) and symmetrical to each other.

The first region (203) comprises: an upper through hole (1) a bottom through hole (5); and a first lower edge (207).

The second region (204) comprises two central upper through holes (2, 3), separated an upper distance (209) from each other.

The third region (205) comprises: an upper through hole (4), a bottom through hole (6) and a second lower edge (207').

The upper through holes (1, 4) are spaced from the bottom through holes (5, 6) a longitudinal distance (206) substantially equal to a distance between the bottom through holes (5, 6) and the lower edges (207, 207').

The through holes (1, 4, 5, 6) are separated a side distance (208) from the attachment means (202).

The upper through holes (1, 4) are separated from the central upper through holes (2, 3), a distance approximately equal to the upper distance (209).

On the other hand, the convex back part of the body (201) comprises some anchoring means for an essentially cylindrical silicone portion of the penis fastening device (100).

As already mentioned, the object of the invention is a system that combines the devices described above. Thus, the system for the penis enlargement object of the invention comprises an essentially annular base intended to rely on a user pelvis and to house a penis inside; comprising two metal arms emanating from the annular base in parallel with each other and longitudinally regarding to the penis, up to a bridge and attachment element between both arms, as the most of standard penis enlargement devices.

However, this system is characterized in that comprises (i) a penis fastening device (100), (ii) a penis support device (200) and constituted as a bridge element between both parallel rods..

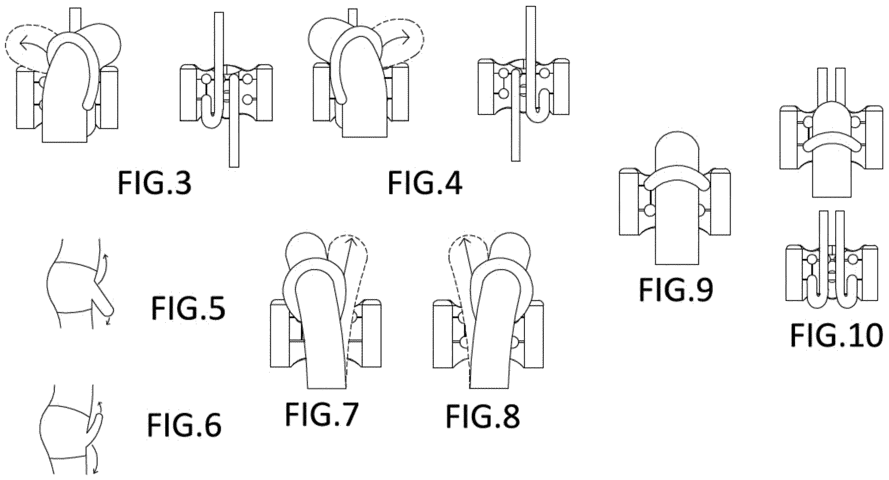

The system thus described, in addition to the enlargement as its general function, is used for the treatment of morphologic disorders of the penis, as shown in the accompanying figures, **FIG. 3** to **FIG. 10**.

In Fig. 3 is showed the curvature correction to the left, wherein the fastening device (100) is anchored to the support device (200) by passing the cylindrical ends of the band, the left end (102a) by the left through hole (2) of the central region (204) of the support device (200), while the right end (102b) passes through the bottom hole (6) of the third region (205) from the body (201) of the support device (200), being anchored both ends to the anchoring means from the rear of the support device (200). Following these operations, the whole system is folded up and is leant to the right.

In Fig. 4 is showed the curvature correction to the right, wherein the fastening device (100) is anchored to the support device (200) by passing the cylindrical ends of the band, the left end (102a) by the right through hole (3) of the central region (204) of the support device (200), while the right end (102b) passes through the bottom hole (5) from first region (203) from the body (201) of the support device (200), being anchored both ends to the anchoring means from the rear of the support device (200). Following these operations, the whole system is folded up and is leant to the left.

In FIGs. 5 and 6 is showed the upward or downward curvature correction. In both cases, the fastening device (100) is introduced through the holes (2, 3) from the central region (204) of the body (201) from the device (200) and the whole system is folded to the opposite side of the curvature.

In Fig. 7 is showed the correction of penile deviation to the left, wherein the fastening device (100) is anchored to the support device (200) by passing the cylindrical ends of the band, the left end (102a) by the top through hole (1) of the first region (203) of the support device (200), while the right end (102b) passes through the right hole (3) from the central region (204) from the body (201) of the support device (200), being anchored both ends to the anchoring means from the rear of the support device (200). Following these operations, the whole system is folded up and is leant to the right.

In Fig. 8 is showed the curvature correction to the right, wherein the fastening device (100) is anchored to the support device (200) by passing the cylindrical ends of the band, the left end (102a) by the left through hole (2) of the central region (204) of the support device (200), while the right end (102b) passes through the top hole (4) from the third region (205) from the body (201) of the support device (200), being anchored both ends to the anchoring means from the rear of the support device (200). Following these operations, the whole system is folded up and is leant to the right.

In FIG. 9 is showed the use of the system to prevent the scar retraction, wherein the penis is protected with a cotton wool or gauze and the cylindrical ends of the fastening device (100) are introduced through the top holes (1, 4) from the first and third regions (203, 205) of the support device (200).

Finally, in FIG. 10 is showed the use of the system for the treatment of penises shorter than 8 cm, wherein the cylindrical ends of the fastening device (100) are introduced through the bottom holes (5, 6) from the first and third regions (203, 205) of the support device (200).

## Claims

1. A penis traction system comprising:
(i) a penis support device (200):
(a) comprising a body (201) made of plastic material; said body (201) also comprising:
(a.1) side attachment means (202); and
(a.2) a concave frontal area defined by three regions (203, 204, 205) wherein:
a first region (203) and a third region (205) are extreme regions in contact with the side attachment means (202) and symmetrical to one another;
the first region (203) comprises:
an upper through hole (1) and a bottom through hole (5);
a first lower edge (207);
the second region (204) comprises two central upper through holes (2, 3), separated an upper distance (209) from each other;
the third region (205) comprises:
an upper through hole (4) and a bottom through hole (6);
a second lower edge (207');
wherein:
the upper through holes (1, 4) are spaced from the bottom through holes (5, 6) a longitudinal distance (206) substantially equal to a distance between the bottom through holes (5, 6) and the lower edges (207, 207');
the through holes (1, 4, 5, 6) are separated a side distance (208) from the attachment means (202);
the upper through holes (1, 4) are separated from the central upper through holes (2, 3), a distance approximately equal to the upper distance (209);
wherein the penis traction system comprises:
a penis fastening device (100):
consisting of a band of flexible, elastic material, having:
a substantially flat central region (101);
substantially cylindrical ends (102a, 102b) having a diameter (x);
wherein the central region (101) has:
a thickness approximately equal to one third of the diameter (x);
a width approximately equal to three times the diameter (x).

2. The system according to claim 1
wherein the central region (101) has a length approximately equal to one fifth of a total length of the penis fastening device (100);
comprising a transition area (103a, 103b) between the cylindrical ends (102a, 102b) and the central region (101).

3. The system according to claim 2, wherein the transition area (103a, 103b) has a length approximately equal to one tenth of a total length of the central region (101).

4. The system according to claims 1 to 3 wherein the material of the penis fastening device (100) is silicone.

5. The system according to claim 3 wherein a convex back part of the body (201) comprises anchoring means for an essentially cylindrical silicone portion of the penis fastening device (100).

6. A multifunctional penile extender comprising:
an essentially annular base:
the base being adapted, in use, to rely on a user pelvis and to house a penis inside; the base comprising metal arms:
the metal arms emanating from the annular base; the metal arms being extended in parallel with each other and longitudinally regarding to the penis, when in use; the penile extender further comprising a system according to any of claims 1 to 5 constituted as a bridge element between the metal arms.

## Patentansprüche

1. Penistraktionssystem umfassend:
(i) eine Penisstützvorrichtung (200):
(a) umfassend einen Körper (201) hergestellt aus Kunststoffmaterial; wobei der genannte Körper (201) auch Folgendes umfasst:
(a.1) seitliche Befestigungsmittel (202); und
(a.2) einen konkaven Stirnbereich, welcher von drei Zonen (203, 204, 205) definiert wird, wobei:
eine erste Zone (203) und eine dritte Zone (205) Endzonen sind, welche in Kontakt mit den seitlichen Befestigungsmitteln (202) und symmetrisch zueinander sind;
die erste Zone (203) Folgendes umfasst:
ein oberes Durchgangsloch (1) und ein unteres Durchgangsloch (5);
einen ersten unteren Rand (207);
die zweite Zone (204) zwei zentrale obere Durchgangslöcher (2, 3) umfasst, welche durch einen oberen Abstand (209) voneinander getrennt sind;
die dritte Zone (205) Folgendes umfasst:
ein oberes Durchgangsloch (4) und ein unteres Durchgangsloch (6);
einen zweiten unteren Rand (207');
wobei:
die oberen Durchgangslöcher (1, 4) von den unteren Durchgangslöchern (5, 6) durch einen Längsabstand (206) getrennt sind, welcher im Wesentlichen einem Abstand zwischen den unteren Durchgangslöchern (5, 6) und den unteren Rändern (207, 207') gleich ist;
die Durchgangslöcher (1, 4, 5, 6) durch einen seitlichen Abstand (208) von den Befestigungsmitteln (202) getrennt sind;
die oberen Durchgangslöcher (1, 4) von den zentralen oberen Löchern (2, 3) getrennt sind, durch einen Abstand, welcher etwa dem oberen Abstand (209) gleich ist;
wobei das Penistraktionssystem Folgendes umfasst:
eine Penisspannvorrichtung (100):
bestehend aus einem Band aus flexiblem, elastischem Material, aufweisend:
eine im Wesentlichen flache zentrale Zone (101);
im Wesentlichen zylindrische Enden (102a, 102b) mit
einem Durchmesser (x);
wobei die zentrale Zone (101) Folgendes aufweist:
eine Dicke, welche etwa einem Drittel des Durchmessers (x) gleich ist;
eine Breite, welche etwa dreimal dem Durchmesser (x) gleich ist.

2. System nach Anspruch 1,
wobei die zentrale Zone (101) eine Länge aufweist, welche etwa einem Fünftel einer Gesamtlänge der Penisspannvorrichtung (100) gleich ist;
umfassend einen Übergangsbereich (103a, 103b) zwischen den zylindrischen Enden (102a, 102b) und der zentralen Zone (101).

3. System nach Anspruch 2, wobei der Übergangsbereich (103a, 103b) eine Länge aufweist, welche etwa einem Zehntel einer Gesamtlänge der zentralen Zone (101) gleich ist.

4. System nach den Ansprüchen 1 bis 3, wobei das Material der Penisspannvorrichtung (100) Silikon ist.

5. System nach Anspruch 3, wobei ein konvexer Rückenteil des Körpers (201) Verankerungsmittel für einen im Wesentlichen zylindrischen Silikonteil der Penisspannvorrichtung (100) umfasst.

6. Multifunktioneller Penisstrecker umfassend:
eine im Wesentlichen ringförmige Basis:
wobei die Basis dazu angepasst ist, im Einsatz, sich auf das Becken eines Benutzers zu stützen und einen Penis darin aufzunehmen;
wobei die Basis Metallarme umfasst:
wobei die Metallarme aus der ringförmigen Basis hervorgeht;
wobei sich die Metallarme, im Einsatz, parallel zueinander und längs in Bezug auf den Penis erstrecken;
wobei der Penisstrecker zusätzlich Folgendes umfasst ein System nach einem der Ansprüche 1 bis 5, welches als Brückenelement zwischen den Metallarmen gebildet ist.

## Revendications

1. Système de traction du pénis comprenant :
(i) un dispositif de support du pénis (200) :
(a) comprenant un corps (201) fabriqué en matière plastique ; ledit corps (201) comprenant en outre :
(a1) des moyens de fixation latérale (202) ; et
(a2) une zone avant concave définie par trois régions (203, 204, 205) dans laquelle :
une première (203) et une troisième région (205) sont des régions extrêmes en contact avec les moyens de fixation latérale (202) et symétriques entre elles ;
la première région (203) comprend :
un trou traversant supérieur (1) et un trou traversant inférieur (5) ;
un premier bord inférieur (207) ;
la deuxième région (204) comprend deux trous traversants supérieurs centraux (2, 3), séparés entre eux par une distance supérieure (209) ;
la troisième région (205) comprend :
un trou traversant supérieur (4) et un trou traversant inférieur (6) ;
un deuxième bord inférieur (207') :
dans lequel :
les trous traversants supérieurs (1, 4) sont séparés des trous traversants inférieurs (5, 6) par une distance longitudinale (206) essentiellement identique à une distance entre les trous traversants inférieurs (5, 6) et les bords inférieurs (207, 207') ;
les trous traversants (1, 4, 5, 6) sont séparés par une distance latérale (208) des moyens de fixation (202) ;
les trous traversants supérieurs (1, 4) sont séparés des trous traversants supérieurs centraux (2, 3) par une distance approximativement identique à la distance supérieure (209) ;
dans lequel le système de traction du pénis comprend :
un dispositif d'attache du pénis (100) :
consistant en une bande de matière élastique flexible, ayant :
une région centrale essentiellement plate (101) ;
des extrémités essentiellement cylindriques (102a, 102b) ayant un diamètre (x) ;
dans lequel la région centrale (101) a :
une épaisseur approximativement identique à un tiers du diamètre (x) ;
une largeur approximativement identique à trois fois le diamètre (x).

2. Système selon la revendication 1,
dans lequel la région centrale (101) a une longueur approximativement identique à un cinquième de la longueur totale du dispositif d'attache du pénis (100) ; comprenant une zone de transition (103a, 103b) entre les extrémités cylindriques (102a, 102b) et la région centrale (101).

3. Système selon la revendication 2, dans lequel la zone de transition (103a, 103b) a une longueur approximativement identique à un dixième de la longueur totale de la région centrale (101).

4. Système selon les revendications 1 à 3, dans lequel la matière du dispositif d'attache du pénis (100) est de la silicone.

5. Système selon la revendication 3, dans lequel une partie arrière convexe du corps (201) comprend des moyens d'ancrage pour une portion de silicone essentiellement cylindrique du dispositif d'attache du pénis (100).

6. Dispositif d'allongement de pénis multifonctionnel comprenant :
une base essentiellement annulaire :
la base étant adaptée, lors de son utilisation, pour être ménagée sur le pelvis de l'utilisateur et pour loger un pénis à l'intérieur de celle-ci ;
la base comprenant des bras métalliques :
les bras métalliques émanant de la base annulaire ;
les bras métalliques étant étendues parallèlement entre eux et longitudinalement par rapport au pénis, lors de leur utilisation ;
le dispositif d'allongement de pénis comprenant en outre :
un système selon l'une quelconque des revendications 1 à 5 constitué comme un élément pont entre les bras métalliques.
